(19) Europäisches Patentamt

European Patent Office

Office européen des brevets

(11) EP 1 413 545 A1

(12) **EUROPEAN PATENT APPLICATION**
published in accordance with Art. 158(3) EPC

(43) Date of publication:
28.04.2004 Bulletin 2004/18

(21) Application number: 02727178.2

(22) Date of filing: 13.05.2002

(51) Int Cl.7: **B82B 3/00**, B01J 19/08,
A61N 1/44, H01J 19/02

(86) International application number:
PCT/CN2002/000328

(87) International publication number:
WO 2003/006362 (23.01.2003 Gazette 2003/04)

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **11.07.2001 CN 01120188**

(71) Applicants:
• **Fang, Moxi**
**Beijing 100086 (CN)**
• **Sun, Yue**
**Beijing 100086 (CN)**

(72) Inventors:
• **Fang, Moxi**
**Beijing 100086 (CN)**
• **Sun, Yue**
**Beijing 100086 (CN)**

(74) Representative: **Schuster, Gregor, Dipl.-Ing.
Patentanwaltskanzlei Schuster,
Wiederholdstrasse 10
70174 Stuttgart (DE)**

(54) **A DEVICE FOR PRODUCING NEGATIVELY CHARGED NANOPARTICLES AND A METHOD FOR THE SAME**

(57) A device and method are provided for producing negatively charged nanoparticles. The device comprises a power supply, an electron supermicroemitter and a controller, the power supply connects with the electron supermicroemitter and the controller respectively. The potential of the electron supermicroemitter to the ground is controlled in the range of -2 kV to -29 kV by the power supply and the controller in accordance with the shape, size and different application of the materials of the emitter, so as to form field electron emitting of tunneling effect. The energy of electrons with high current density produced by the emitter can be adjusted during the electrons' colliding with particles in aerosol such that the electrons are attached to the nanoparticles of different size with wider energy band to form negatively charged nanoparticles.

**Fig. 1**

EP 1 413 545 A1

## Description

### FIELD OF THE INVENTION

The present invention relates to a device and method for producing nanoparticles, specifically to a device and method for producing novel negatively charged nanoparticles by combining two particles and electric charges in bioclimatology and physics, which are unrelated with each other, to be used in fields of medicine, home appliance, aseptic engineering, freshness preservation engineering, bioengineering, and the like.

### BACKGROUND OF THE INVENTION

In bioclimatology, the state of air environmental condition is called by scientific workers as the aerosol state. Molecule cluster, liquid and solid particles dispersed in air as aerosols are mostly nanoparticles.

Nanoparticles exhibit small-scale effect, surface and interfacial effect and quantum-scale effect, and have large specific surface area and big number of atoms on the surface. Surface effect and interfacial effect are intensified with decreasing particle size. The big specific surface area and the big number of atoms on the surface increase the activity of the nanoparticle greatly. Due to the small-scale effect and surface effect, nanoparticles of different size also cause variation of surface electron spin conformation and electron energy spectra distribution. Quantum-scale effect of nanoparticles results in discrete energy level. The interval between energy levels changes with the changes of the nanoparticle sizes. Nanoparticles in aerosol are composed of different particles with different sizes. Because of the above-mentioned properties, nanoparticles greatly enhanced the ability to combine with electrons of different energy levels to form a very wide energy band of electron affinity.

The problem is what kind of electron-emitting electrode can be used to achieve a strong enough electric field with narrowing potential barrier on the electrode surface. Due to the tunnel effect in quantum mechanics, electrons will penetrate and escape from the tunnel as field electron emission. How to increase the emission current density is a problem under research.

In the 1960s, electrically charged aerosol centers were established in Texas and other states in the US. By ejecting pressurized gas the atomized physiological saline and electrons were emitted at the same time in the same direction at an electric potential of 26 kV-60 kV on an ejector to form electrically charged aerosol. Such aerosol was used to cure respiratory disease. It was effective in curing bronchitis and asthma, infection of the upper respiratory tract, emphysema, laryngitis, and pharyngitis. Besides the ejector, auxiliary equipments of gas pump, and liquid transport system were required. Atomized saline particles were mostly not nanoparticles. Even under the action of 26 kV-60 kV electric field, electrically charged aerosol could only travel a distance of about 1.8 m and disappeared beyond that distance. Such electrically charged aerosol was not able to directly participate in the electric metabolism at the tissue-cell-molecule level, so the biological effect and sterilizing effect were less promising.

### DISCLOSURE OF THE INVENTION

The object of the present invention is to adopt an electron supermicroemitter at a micron-level or sub micron-level to provide very high emitter current density. When the electrode surface has a strong enough electric field, the potential barrier of electrode will narrow and electrons on the electrode will penetrate and escape from the tunnel because of tunnel effect in quantum mechanics to form field emission electrons, which can provide a very high emission current density.

The present invention is to combine the physical characteristics of nanoparticles and the tunnel effect in quantum mechanics. When the electrons 'e' emitted by the electron emitting electrode collide with the particles in aerosol, the electrons can adjust the energy and adhere to the nanoparticles 'Nm' with a broad energy band of electron affinity to form new negatively charged nanoparticles 'N⁻m', that is to realize: $e + Nm \rightarrow N^-m$.

Generally, there exist a few particles with different electric charges in air. Particles with and without electric charge can attract with each other and coalesce, resulting in combination of opposite charges and fall-off in large particles with the electric charge disappearing upon contact with ground. The negatively charged nanoparticles produced by the present invention appear in a large amount in a certain scope with same electric charges repelling each other. Scientists of bioclimatology and physics all think that such state of system is more stable.

Such novel particles produced by using the physical characteristics and tunnel effect in quantum mechanics of nanoparticles inevitably leads to exclusively negatively charged nanoparticles without the presence of any other compounds or impurities.

The device for producing negatively charged nanoparticles of the present invention comprises a power supply, a casing, a controller and an electron supermicroemitter, wherein, the power supply connects with the electron supermicroemitter and the controller respectively, and the potential of the electron supermicroemitter to the ground is controlled in the range of - 2 kV to - 29kV.

The said electron supermicroemitters are those with an electrode of an emitting body having a dimension at a micron level or sub-micron level. The material for preparing the said electron supermicroemitter of the present invention is platinum, gold, rhenium, iridium, tungsten or carbon fiber or their combination, or alloys with platinum, gold, rhenium, iridium and/or tungsten as the main components. The shape of the electrode could be any one or combination of the shapes selected from the group consisting of disk, cylinder, saw teeth, needle, sharp-ended, sphere, spheroid, arc, ring, bar, etc. The electron supermicroemitter could be a single electrode or multiple electrodes. The dimension of the electron supermicroemitter is ≤100 micron.

The method for producing negatively charged nanoparticles according to the present invention is as follows. The negatively charged nanoparticles producing device constructed by connecting the power supply with the electron supermicroemitter and the controller respectively is used. The potential of the nanoparticles in air and the electron supermicroemitter to the ground, under the action of the power supply and the controller, are controlled in the range of - 2 kV to - 29 kV. Electrons emitted by tunnel effect combines with the nanoparticles to produce new negatively charged nanoparticles. The electric potential range is determined by the material, shape, and dimension of the electrode and the different application equipment as used.

Field emission by tunnel effect generates electrons 'e' of high electric current density, which upon colliding with particles in aerosol, can adjust the energy (for example, electrons of high energy can lose its energy or reduce its energy on collision) and adhere on nanoparticles 'Nm' of different sizes with a broad energy band (The nanoparticles in air consist of various molecule clusters--either in the solid state, liquid state or gaseous state--and nanoparticles of different sizes ($10^{-7}$-$10^{-9}$ m).). The reaction is as follows:

$$e + Nm \rightarrow N^-m$$

Negatively charged nanoparticles are thereby produced. Under the action of electric field at any potential in the range of -2 kV to -29 kV, these particles can rapidly diffuse outward to cover a certain area.

The said electron supermicroemitter can be made according to one of the following methods:

a) Platinum, gold, or carbon fiber filament is fixed on a glass carriage by a soldering method. The leading-out end is made by bonding platinum, gold, or carbon fiber to copper wire with conducting glue (such as a conducting glue made of silver powder and epoxy resin). Platinum wire can be connected to the conductor with indium melted at a low temperature.

b) Platinum, gold, rhenium, tungsten, iridium or carbon fiber filament is bonded and sealed in a carriage made of insulators made of quartz, glass, PE, PTFE (plastics), polyester fiber, silicon nitride, alumina (porcelain) with epoxy resin adhesive. The leading-out end is made by bonding platinum, gold, rhenium, tungsten, iridium or carbon fiber filament to copper wire with conducting glue (such as a conducting glue made of silver powder and epoxy resin). Fixation of the leading-end and the conductor can follow the same procedures as mentioned in method "a".

c) Platinum, gold, rhenium, tungsten, iridium or carbon fiber filament is arranged on the surface of an insulator in such shapes as bar, ring, arc, etc. It is then fixed and bonded with adhesive, such as epoxy resin. Fixation of the insulator, the leading-out end and the conductor can follow the same procedures as mentioned in methods "a" and "b".

d) Rhenium, tungsten or its corresponding alloy are made into electrodes of various shapes by electrolytic corrosion, such as the sharp-ended shape, needle shape, saw teeth shape, etc. In regard to the electrolytic corrosion, it has been taught in various common textbooks or literatures. The electrode made hereby is fixed on an insulator carriage with epoxy resin or riveted on an insulator carriage. According to the method of fixation, the insulator can be quartz, glass, PE, PTFE (plastics), silicon nitride, alumina (porcelain), polyester composite plate, etc. The leading-out end can be bonded to the conductor with conducting glue, or the leading-out wire and the electrode can be fixed on an insulator at the same time by mechanical means. The connection is illustrated by the following two examples. There are options for fixing and leading-out of the saw-teeth shaped electrode. One is the use of an epoxy resin and conducting glue. Saw teeth electrode is bonded with an epoxy resin to the insulator located underneath with one side of electrode connected to leading-out wire with conducting glue. The other is mechanical means. According to the mechanical means, saw teeth electrode is fastened with rivets on an insulator by clamps on both sides and the leading-out wire is riveted with a clamp on one side of the electrode.

The sharp-ended and needle electrode can also use the above options except for some specific structure. For example, fixing clamp is not required for the needle electrode. A pin sleeve or rivet sleeve is used to fasten the electrode and lead wire directly on the insulator.

e) Photoetching can be used to make electron supermicroemitter. According to this method, a uniform metallic film is coated on an insulator plate by spraying or sputtering. The metal film can be made of platinum, gold, iridium, etc. A photosensitive polymer film of polyimide is coated on the metallic film, and photoetching is carried out to form an electrode with a required shape. The matrix material of the electrode can be $Si/SiO_2$, quartz, glass, silicon nitride, etc. Leading-out wire is made by bonding the electrode to copper wire with conducting glue.

The sizes of the negatively charged nanoparticles are smaller than those of red blood cells in blood and ordinary bacteria, and are a fraction of the latter or even much smaller. Such nanoparticles can enter human body through respiration and skin mucous membrane into the lung and blood circulation and release electric charge. The equilibrium state of electric charges on cell wall can be improved to form bio-electricity having direct biological effect on physiological condition, tissue cells and metabolism of human body.

Negatively charged nanoparticles in human body can directly participate in electro-metabolism at tissue-cell-molecule level, promoting transform of bio-electricity, adjusting electric potential equilibrium of organism, and improving natural physiological condition and biochemical environment of human body. The non-specific and broad-spectrum medical effect is achieved through the adjusting function of nerve-humor.

Negatively charged nanoparticles with apparent biological effect have apparent conditioning opsonic function on neural system, cardio-vascular system, respiratory system, urinary system and digestive system and have curative effect on many diseases. Clinical application of the negatively charged nanoparticles has caught wide attention.

Experimental studies demonstrate that, due to the quantum mechanics characteristics, the negatively charged nanoparticles can rapidly cover a certain scope and strongly inhibit the growth of and eradicate such harmful bacteria and viruses as Pseudomonas aeruginosa, syphilis spirochete, staphylococcus, bacillus coli, mycosis, Monilia, etc.

It is well known that research and application of bioelectricity have been developing continuously. Clinical application of electroencephalogram (EEG), electrocardiogram (ECG), electrogastrogram (EGG) has saved the lives of countless people. Just like the clinical application of physical medical diagnosis equipment, the computerized tomography (CT), brightness type ultrasonic diagnostic apparatus, nuclear magnetic resonance apparatus, and positive electron tomography have opened a new era for the uninterrupted development of clinical diagnosis. Bioelectricity of negatively charged nanoparticles will create many new techniques and new equipment, especially in clinical treatment.

Therefore, bioelectricity of negatively charged nanoparticles can be widely used in physical medical equipment, home appliance, bioengineering, freshness preservation engineering, aseptic engineering and environmental condition improvements.

**BRIEF DESCRIPTION OF DRAWINGS**

Figure 1 is the block diagram showing the principle of the present invention.

Figure 2 is the schematic drawing of the leading-out for the saw teeth electrode bonded with epoxy resin and conducting glue.

Figure 3 is the schematic drawing of the leading-out for the saw teeth electrode fastened with mechanical means.

**BEST MODE FOR CARRYING OUT THE INVENTION**

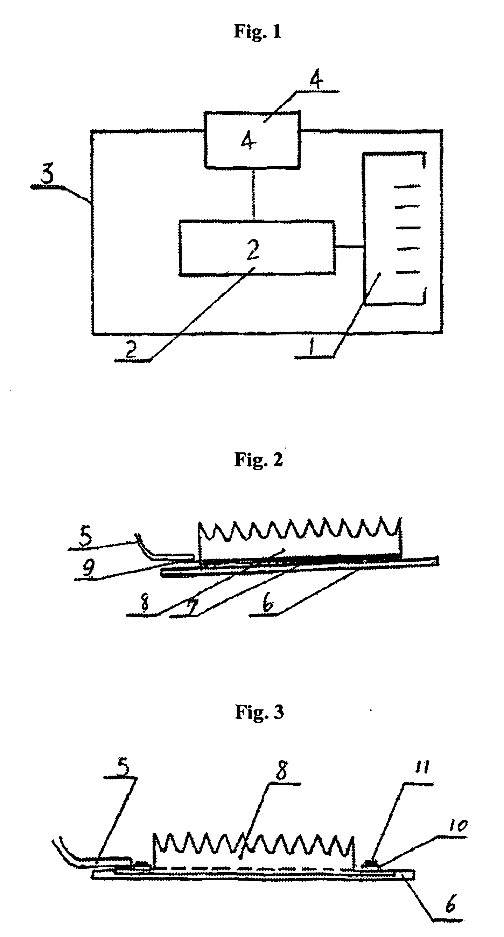

As shown in Figure 1, the device of the present invention comprises basic component parts of an electron supermicroemitter 1, a power supply 2, a casing 3, and a controller 4. Other parts can be added according to the usage and device, such as multi-functions carriage or turntable. The present invention can also be combined with other device to form a new equipment with new functions, with its control device compatible with other parts of the control equipment.

According to the purpose of application and the function of product, the electron supermicroemitter can be a single electrode, multiple electrodes or a combination electrode. The device casing can be designed as one with totally different shape, function and configuration, as the potential of the electron supermicroemitter to the ground is controlled in the range of -2 kV to -29 kV, depending on its structure, dimension, shape and material, and purpose of application.

As shown in Figure 2, epoxy resin and conducting glue are used to fix and leading-out the electrode. Saw teeth electrode 8 is bonded underneath to the insulator 6 by epoxy resin 7, and leading-out wire is made of a conductor 5 bonded to one end of the electrode 8 with conducting glue 9.

As shown in Figure 3, mechanical means is used to fasten and leading-out the electrode. Saw teeth electrode 8 is fastened with an insulator 6 by a clamp 10 and a rivet 11. Leading-out wire 5 is fastened with the clamp 10 and the rivet 11.

**Claims**

1. A device for producing negatively charged nanoparticles, comprising a power supply, a casing, a controller and an electron supermicroemitter, **characterized in that** the power supply connects with the electron supermicroemitter and the controller respectively, the potential of the electron supermicroemitter to the ground is controlled in the range of -2 kV to -29 kV.

2. The device as claimed in Claim 1, **characterized in that** the electron supermicroemitter is comprised of a single or multiple electrodes, the shape of the electrode is any one or combination of the shapes selected from the group consisting of disk, cylinder, saw teeth, needle, sharp-ended, sphere, spheroid, arc, ring, bar, and the size of the emission part of the electron supermicroemitter is ≤100 micron.

3. The device as claimed in Claim 1 or Claim 2, **characterized in that** the electron supermicroemitter is made of platinum, gold, rhenium, iridium, tungsten or carbon fiber or their combination or an alloy with platinum, gold, rhenium, iridium and/or tungsten as the main component.

4. A method for producing negatively charged nanoparticles, **characterized in that**, the device of claim 1 is used to control the potential of the electron supermicroemitter to the ground, under the action of the power supply and the controller, in the range of -2 kV to -29 kV depending on the electrode material, electrode shape, electrode dimension and different application equipment as used; field electron emission by tunnel effect generates electrons 'e' of high electric current density, which upon colliding with particles in aerosol, can adjust the energy and adhere on nanoparticles 'Nm' of different sizes with a broader energy band of electron affinity; the reaction is as follows:

$$e + Nm \rightarrow N^-m$$

and negatively charge nanoparticles are produced thereby.

5. The method as claimed in Claim 4, **characterized in that** the electron supermicroemitter is made according to one of the following methods:

a) Platinum, gold, or carbon fiber filament is fixed on a glass carriage by a soldering method, the leading-out end is made by bonding platinum, gold, or carbon fiber filament to copper wire with conducting glue, platinum wire can also be connected with copper wire by indium melted at a low temperature;

b) Platinum, gold, rhenium, tungsten, iridium or carbon fiber filament is bonded and sealed in a carriage made of insulators of quartz, glass, PE, PTFE, polyester fiber, silicon nitride, and/or alumina (porcelain) with epoxy resin adhesive, the leading-out end is made by bonding platinum, gold, rhenium, tungsten, iridium or carbon fiber to copper wire with conducting glue;

c) Platinum, gold, rhenium, tungsten, iridium or carbon fiber filament is arranged on the surface of an insulator made of quartz, glass, PE, PTFE, polyester fiber, silicon nitride and/or alumina in a required shape, it is then fixed and bonded with adhesive, platinum, gold, rhenium, tungsten, iridium or carbon filament is bonded to copper wire with conducting glue as leading-out end;

d) Rhenium, tungsten or their corresponding alloy is made into electron supermicroemitters of various shapes by electrolytic corrosion, the said electron supermicroemitter is fixed on an insulator carriage with epoxy resin or riveted on insulator carriage by mechanical means, the insulator can be any one of quartz, glass, PE, PTFE, silicon nitride, alumina, polyester composite plate, the leading-out end can be bonded to conductor with conducting glue, or the lead conductor and electrode can be fixed on insulator at the same time by mechanical means, such method likewise applies to the sharp-ended and needle electrode; or

e) Photoetching is utilized to make electron supermicroemitter: a uniform metallic film is coated on an insulator plate by spraying or sputtering, the metal can be platinum, gold, iridium, a photosensitive polymer film of polyimide is coated the metallic film, and photoetching is carried out to form electrode of the required shape, the matrix material of the electrode can be any one of Si/SiO$_2$, quartz, glass, silicon nitride, leading-out wire is made by bonding electrode to copper wire with conducting glue.

**Fig. 1**

**Fig. 2**

**Fig. 3**

**EP 1 413 545 A1**

# INTERNATIONAL SEARCH REPORT

International application No.

PCT/CN02/00328

| A. CLASSIFICATION OF SUBJECT MATTER |
|---|
| IPC[7]: B82B3/00 A61N1/44, B01J19/08 H01J19/02 |
| According to International Patent Classification (IPC) or to both national classification and IPC |

| B. FIELDS SEARCHED |
|---|
| Minimum documentation searched (classification system followed by classification symbols) |
| IPC[7]: A61N1/44, 1/00 B82B1/00, 3/00 G01N13/10, 13/12 B01J19/00, 19/08 B01B5/00, 5/025, 5/03 H01J19/02, 19/066, 19/42, 19/60, 19/62 B03C3/38, 3/40, 3/43 |
| Documentation searched other than minimum documentation to the extent that such documents are included in the fields searched |
| Electronic data base consulted during the international search (name of data base and, where practicable, search terms used) |
| Chinese application searching system : nanoparticle microparticle electron electrode 或 electron w emitter EPODOC & WPI & PAJ: charg+ and +particle+ electron electrode or emitter |

| C. DOCUMENTS CONSIDERED TO BE RELEVANT |
|---|

| Category* | Citation of document, with indication, where appropriate, of the relevant passages | Relevant to claim No. |
|---|---|---|
| X/Y | US 5247842 A （TSI Incorporated） 28.Sep.1993 (28.12.1993) See the description column 5 line 7 ~ column 9 line 47 | 1,2/3,4 |
| Y | US 4954711 A (International Business Machines Corporation) 04.Sep.1990(04.09.1990) See the description column 1 line 41 行 | 3 |
| Y | JP 2-159283 A (SHIGECHIKA YAMAGISHI et. al) 19.Jun.1990 (19.06.1990) See the description page 2 embodiment 2 | 4 |

| ☐ Further documents are listed in the continuation of Box C. ☒ See patent family annex. |
|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13.Agu.2002 （13.08.2002） | 2 2 AUG 2002 |
| Name and mailing address of the ISA/CN | Authorized officer |
| 6 Xitucheng Rd., Jimen Bridge, Haidian District, 100088 Beijing, China | Meng Haiyan |
| Facsimile No. 86-010-62019451 | Telephone No. 86-010-6209394 |

Form PCT/ISA /210 (second sheet) (July. 1998)

## INTERNATIONAL SEARCH REPORT

International application No.

PCT/CN02/00328

| C (Continuation). DOCUMENTS CONSIDERED TO BE RELEVANT | | |
|---|---|---|
| Category* | Citation of document, with indication, where appropriate, of the relevant passages | Relevant to claim No. |
| A | CN 1107373 A （Guangxi Institute of medical sciences） 30.Aug.1995 (30.08.1995) <br><br> See the description page 1 line 9 ~ page 3 line 18 | 1~4 |

Form PCT/ISA /210 (continuation of second sheet (1)) (July. 1998)

## INTERNATIONAL SEARCH REPORT
### Information on patent family members

| International application No. |
| --- |
| PCT/CN02/00328 |

| Patent document Cited in search report | Publication Date | Patent family Member(s) | Publication Date |
| --- | --- | --- | --- |
| US 5247842 A | 28.09.1993 | WO9307465A | 15.04.1993 |
| | | EP0746751AB | 28.09.1993 |
| | | DE69228953D | 20.05.1999 |
| | | DE69228953T | 07.10.0999 |
| US 4954711 A | 04.09.1990 | EP0366851AB | 09.05.1990 |
| | | JP2186545A | 20.07.1990 |
| | | JP6056745B | 27.07.1994 |
| | | JP1926196C | 25.04.1995 |
| | | CA1311863A | 22.12.1992 |
| | | DE3887891D | 24.03.1994 |
| | | DE3887891T | 11.08.1994 |
| JP 2-159283 A | 19.06.1990 | DE3915907A | 21.06.1990 |
| CN 1107373 A | 30.08.1995 | None | |

Form PCT/ISA /210 (patent family annex) (July 1998)

9